# EUROPÄISCHE PATENTSCHRIFT

(11) **EP 2 381 996 B1**
(45) Veröffentlichungstag und Bekanntmachung des Hinweises auf die Patenterteilung: **30.07.2014**
(21) Anmeldenummer: 10700369.1
(22) Anmeldetag: 07.01.2010
(51) Int. Cl.: A61M 16/12

(54) **VORRICHTUNG ZUM VERSORGEN EINES PATIENTEN MIT EINEM GAS**
DEVICE FOR PROVIDING A GAS TO A PATIENT
DISPOSITIF D'ADMINISTRATION D'UN GAZ À UN PATIENT

(30) Priorität: 08.01.2009 DE 102009004107
(43) Veröffentlichungstag der Anmeldung: 02.11.2011
(73) Patentinhaber: Linde AG, 80331 München (DE)
(72) Erfinder: MÜLLER, Rainer, A-2351 Wiener Neudorf (AT); ROMAKO, Christian, A-3382 Loosdorf (AT); KÖHLE, Christian, A-2120 Wolkersdorf im Weinviertel (AT)
(86) Internationale Anmeldenummer: PCT/EP2010/000048
(87) Internationale Veröffentlichungsnummer: WO 2010/079134

(56) Entgegenhaltungen:
- EP-A1- 0 861 672
- EP-A2- 0 894 506
- DE-A1- 19 639 522
- DE-B3-102004 052 398
- DE-U1- 9 218 160
- FR-A1- 2 862 227
- FR-A1- 2 894 486
- GB-A- 2 368 531

## Beschreibung

Die Erfindung betrifft eine Vorrichtung zum Versorgen eines Patienten, insbesondere eines spontanatmenden Patienten, mit einem Gas.

Unter dem Begriff "Gas" seien nachfolgend Gase und Gasgemische zu verstehen.

Eine Vorrichtung zum Versorgen eines spontanatmenden Patienten mit einem Gas ist bspw.aus der EP-A 0861672 bekannt. Dieses Dokument offenbart eine Vorrichtung gemäß dem Oberbegriff des Anspruchs 1. Derartige Vorrichtungen kommen bspw. bei der nichtinvasiven Beatmung von spontanatmenden Patienten mittels des CPAP-Verfahrens (Continuous Positive Airway Pressure) zur Anwendung. Hierbei wird im Regelfall ein konstanter Gasdurchfluss realisiert. Der Durchfluss wird dabei so eingestellt, dass der notwendige Unterstützungsdruck während der Inhalations- sowie der Exhalationsphase erreicht wird. Üblicherweise erfolgt die Zuführung des Gases zu dem Patienten mittels einer Nasalmaske, vorzugsweise mittels einer Gesichtsmaske. Ferner können gattungsgemäße Vorrichtungen bei intubierten Patienten Verwendung finden.

Bei einer Vielzahl von Inhalations-Therapien werden Gase verwendet, die entweder nur begrenzt zur Verfügung stehen - bspw. im Falle der Bereitstellung der Gase mittels Druckgasflaschen - oder die bei zu hohen Konzentrationen innerhalb des Behandlungsraumes gesundheitsschädlich sein können; dies ist bspw. bei Lachgas (N₂O) der Fall. Lachgas wird mit Sauerstoff vermischt und spontanatmenden Patienten zwecks analgetischer Wirkung verabreicht.

Derartige Therapien werden üblicherweise in Behandlungsräumen praktiziert, die keine, der Absaugung exhalierter Gase dienenden Einrichtungen aufweisen. Ein Absaugen der Gase würde verhindern, dass exhalierte Gase in den Raum gelangen und damit die Gas- bzw. Arbeitsplatzkonzentration in dem Raum über den bzw. die zulässigen Grenzwerte erhöhen.

Des Weiteren ist zu beachten, dass der Patient nur einen Bruchteil der inhalierten Gase im Körper aufnimmt, während ein großer Anteil der inhalierten Gase wieder ungenutzt exhaliert wird. Dies führt dazu, dass selbst im Falle einer kurzen Therapiedauer die erlaubten Arbeitsplatzkonzentrationen überschritten werden können.

Ein weiteres Problem ergibt sich bei der Verwendung von Gasen, die ausschließlich in Druckgasflaschen bereitgestellt werden; beispielhaft genannt hierfür seien Helium-Sauerstoffgemische. Derartige Gase sind vergleichsweise teuer und werden nicht über das zentrale Gasversorgungssystem des Krankenhauses zur Verfügung gestellt. Dies bedeutet, dass diese Gase ausschließlich in Druckgasflaschen unterschiedlicher Größen zur Verfügung stehen. Insbesondere die Therapie mit Helium-Sauerstoffgemischen kann jedoch mehrere Stunden andauern. Dies führt dazu, dass die Gasflaschen sehr oft während der Therapie ausgetauscht werden müssen. Der mit dem Austausch der Gasflaschen verbundene logistische Aufwand ist u. U. erheblich. Dies wiederum führt zu einem negativen Einfluss auf die Akzeptanz derartiger Therapien.

Aufgabe der vorliegenden Erfindung ist es, eine gattungsgemäße Vorrichtung zum Versorgen eines Patienten, insbesondere eines spontanatmenden Patienten, mit einem Gas anzugeben, die die vorbeschriebenen Nachteile vermeidet und insbesondere eine Optimierung des Gasverbrauchs bei unterschiedlichsten Inhalations-Therapien spontanatmender Patienten ermöglicht. Hierbei sollen Therapieparameter, die die Compliance des Patienten gegenüber der Therapie beeinflussen können, wie der inhalative und der exhalative Atemwiderstand, nicht nennenswert beeinflusst werden.

Zur Lösung dieser Aufgabe wird eine Vorrichtung gemäß dem Anspruch 1 angegeben.

Weitere vorteilhafte Ausgestaltungen der erfindungsgemäßen Vorrichtung sind Gegenstand der abhängigen Ansprüche.

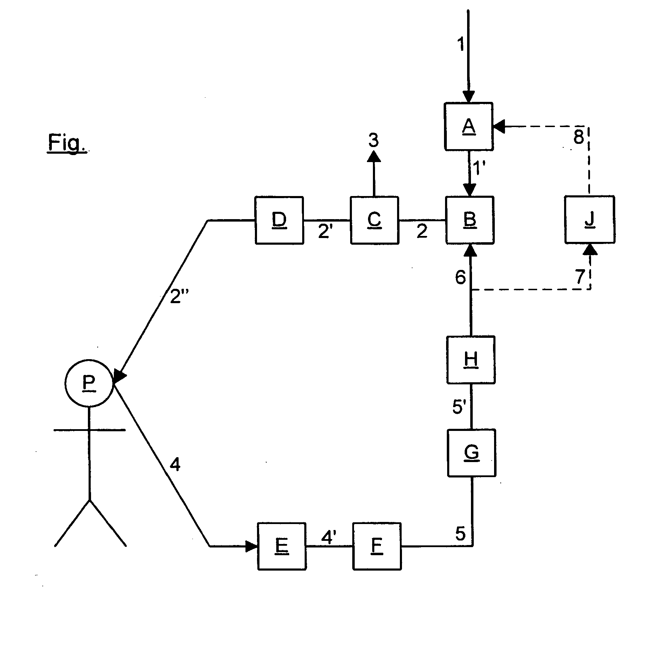

Die erfindungsgemäße Vorrichtung zum Versorgen eines (spontanatmenden) Patienten mit einem Gas sowie weitere Ausgestaltungen derselben seien nachfolgend anhand des in der Figur dargestellten Ausführungsbeispieles näher erläutert.

Die erfindungsgemäße Vorrichtung zum Versorgen eines (spontanatmenden) Patienten mit einem Gas weist zumindest die nachfolgend genannten Bestandteile auf: eine Dosiervorrichtung A, einen Gasspeicher B, ein Demand-Ventil D, ein Reservoir F, eine Kohlendioxid-Abtrenneinheit G, eine Pumpe H sowie eine Analyse/Steuereinheit J. Des Weiteren können ein Einwege-Ventil E und eine Spülvorrichtung C vorgesehen sein.

Der spontanatmende Patient P atmet während der Inhalationsphase über das Demand-Ventil D sowie die Leitungsabschnitte 2 - 2" die benötigte Menge an Gas, das im Gasspeicher B bereitgestellt ist, ein. Innerhalb des Gasspeichers B herrscht ein Überdruck von bspw. > 500 mbar. Die Zusammensetzung des in dem Gasspeicher B bereitgestellten Gases muss in Abhängigkeit von der gewählten Therapieart während der Therapiedauer konstant gehalten werden.

Mittels des Demand-Ventils D wird der innerhalb des Gasspeichers B herrschende Überdruck auf den durch den Patienten P bestimmten Inhalationsdruck reduziert. Die Gasmenge, die durch das Demand-Ventil D fließt, wird durch den Patienten P gesteuert. Somit wird sichergestellt, dass exakt die Gasmenge, die vom Patienten aufgrund der gewählten Therapieart benötigt wird, zur Verfügung gestellt werden kann.

Das vom Patienten P ausgeatmete Gas wird über die Leitungsabschnitte 4 und 4' einem Reservoir F zugeführt. Dieses Reservoir F ist vorzugsweise als Atembeutel bzw. Bag ausgebildet. Die Zusammensetzung des ausgeatmeten Gases unterscheidet sich im Regelfall von der Zusammensetzung des inhalierten Gases. Das Reservoir F dient der Zwischenspeicherung des ausgeatmeten Gases. Es ist vorzugsweise derart ausgebildet, dass bei Vergrößerung des Gasvolumens innerhalb des Reservoirs F der Druck nur geringfügig ansteigt. Dies ermöglicht eine minimale Atemanstrengung für den Patienten P bei der Exhalation. Soll verhindert werden, dass der Patient P während der Inhalation aus dem Reservoir F zurückatmet, empfiehlt es sich, dem Reservoir E ein Einwege-Ventil E vorzuschalten. Im Regelfall ist ein derartiges Einwege-Ventil E zwingend vorzusehen, da der Patient nicht aus dem Reservoir F zurückatmen darf.

Das aus dem Reservoir F abgeführte bzw. abgezogene Gas wird über Leitung 5 einer Kohlendioxid-Abtrenneinheit G zugeführt. Mittels dieser wird das vom Patienten P exhalierte Kohlendioxid aus dem Gas entfernt. Die Kohlendioxid-Abtrenneinheit G ist als eine absorptiv, adsorptiv und/oder permeativ arbeitende Abtrenneinheit ausgebildet. Das Zuführen des aus dem Reservoir F abgezogenen Gases über Leitung 5 erfolgt durch die der Kohlendioxid-Abtrenneinheit G nachgeschalteten Pumpe H.

Diese fördert das Gas über die Leitungsabschnitte 5' und 6 anschließend in den bereits beschriebenen Gasspeicher B. Mittels der Pumpe H wird das Druckniveau von dem Druckniveau des Patienten P - dieses liegt nahe Umgebungsdruck - auf das Arbeitsdruckniveaus des vorbeschriebenen Demand-Ventils D erhöht. Durch das Vorsehen einer Drucksteuerung kann die Pumpe H bei geleertem Reservoir F abgeschaltet werden.

Mittels der Analyse/Steuereinheit J wird die Zusammensetzung des über Leitung 6 zurückgepumpten Gases bestimmt - in der Figur dargestellt durch die gestrichelt gezeichnete Leitung 7. Entspricht die analysierte Gaszusammensetzung nicht den Vorgaben, erfolgt über die gestrichelt gezeichnete Leitung 8 eine Ansteuerung der Dosiervorrichtung A.

Mittels der Dosiervorrichtung A kann bzw. können diejenige(n) Komponente(n) des in dem Gasspeicher B bereitgestellten Gases, deren Gehalt in der ermittelten Gaszusammensetzung zu niedrig ist, über die Leitungsabschnitte 1 und 1' dem Gasspeicher B zudosiert werden. Die Dosiervorrichtung A vergleicht hierzu die Ist- mit der Sollkonzentration und regelt anhand der berechneten Abweichung die erforderliche(n) Gasmenge(n), die notwendig sind, um die Sollkonzentration in dem Gasspeicher B zu erreichen. Somit kann die Zusammensetzung des vom Patienten P eingeatmeten Gases konstant gehalten werden. Die Leitung 1 ist mit einer in der Figur nicht dargestellten, beliebig ausgebildeten Gasquelle (Druckgasflasche, Flüssigtank, zentrales Gasversorgungssystem eines Krankenhauses, etc.) verbunden bzw. verbindbar.

Des Weiteren kann eine Spülvorrichtung C vorgesehen werden. Über diese bzw. deren Ablassleitung 3 kann (überschüssiges) Gas aus dem System abgelassen werden, um die Gasmenge in dem Gasspeicher B beeinflussen zu können.

Beim nächsten Atemzug inhaliert der Patient P erneut über das Demand-Ventil D das auf die Sollkonzentration geregelte Gas(gemisch) aus dem Gasspeicher B. Die Größe des Gasspeichers B ist so auszulegen, dass dem Patienten P während der Inhalation immer ausreichend Gas zur Verfügung gestellt werden kann.

Die erfindungsgemäße Vorrichtung zum Versorgen eines (spontanatmenden) Patienten mit einem Gas weist gegenüber den zum Stand der Technik zählenden Vorrichtungen folgende Vorteile auf:
- Geringer Atemwiderstand beim Ein- und Ausatmen
- Vereinfachte Gasanalyse durch konstanten Pumpenflow
- Geringer Gasverbrauch durch geschlossenen Kreislauf; mittels der Dosiervorrichtung werden nur die Gasverluste ausgeglichen
- Geschlossener Kreislauf verringert Belastung der Umgebung mit Atemgasen; die zulässigen Arbeitsplatzkonzentrationen können einfacher eingehalten werden
- Keine Gasverluste beim Abnehmen der Patienten-Maske aufgrund des Demand-Ventils

## Patentansprüche

1. Vorrichtung zum Versorgen eines Patienten, insbesondere eines spontanatmenden Patienten, mit einem Gas, aufweisend
- eine Dosiervorrichtung (A), die der Dosierung des von dem Patienten (P) zu inhalierenden Gases in den Gasspeicher (B) dient,
- einen Gasspeicher (B), der der Speicherung des von dem Patienten (P) zu inhalierenden Gases dient,
- ein Demand-Ventil (D), das den Mengenfluss des von dem Patienten (P) inhalierten Gases regelt, und
- eine Kohlendioxid-Abtrenneinheit (G), die der Abtrennung von Kohlendioxid aus dem ausgeatmeten Gas dient,
**dadurch gekennzeichnet, dass**
- ein Reservoir (F), das der Aufnahme des von dem Patienten (P) ausgeatmeten Gases dient,
- eine Pumpe (H), die der Rückführung des ausgeatmeten, von Kohlendioxid gereinigten Gases in den Gasspeicher (B) dient, und
- eine Analyse/Steuereinheit (J), die das in den Gasspeicher (B) zurückgeführte Gas analysiert und in Abhängigkeit von dessen Zusammensetzung die Dosiervorrichtung (A) ansteuert, vorgesehen sind.

2. Vorrichtung nach Anspruch 1, **dadurch gekennzeichnet, dass** dem Reservoir (F) ein Einwege-Ventil (E), das ein Rückströmen des von dem Patienten (P) ausgeatmeten Gases verhindert, vorgeschaltet ist.

3. Vorrichtung nach Anspruch 2, **dadurch gekennzeichnet, dass** die Kohlendioxid-Abtrenneinheit (G) als eine absorptiv, adsorptiv und/oder permeativ arbeitende Kohlendioxid-Abtrenneinheit ausgebildet ist.

4. Vorrichtung nach einem der vorhergehenden Ansprüche 1 bis 3, **dadurch gekennzeichnet, dass** dem Demand-Ventil (D) eine Spülvorrichtung (C), die der Abführung von Gas dient, vorgeschaltet ist.

5. Vorrichtung nach einem der vorhergehenden Ansprüche 1 bis 4, **dadurch gekennzeichnet, dass** das Reservoir (F) als Atembeutel ausgebildet ist.

6. Vorrichtung nach einem der vorhergehenden Ansprüche 1 bis 5, **dadurch gekennzeichnet, dass** die Pumpe (H) eine Sicherungschaltung, die ein vollständiges Entleeren des Reservoirs (F) verhindert, aufweist.

## Claims

1. Device for supplying a patient, in particular a spontaneously breathing patient, with a gas, having
- a metering device (A), which serves for metering the gas that is to be inhaled by the patient (P) into the gas storage unit (B),
- a gas storage unit (B), which serves for storing the gas to be inhaled by the patient (P),
- a demand valve (D), which regulates the amount of flow of the gas inhaled by the patient (P), and
- a carbon dioxide separating unit (G), which serves for separating carbon dioxide from the exhaled gas,
**characterized in that**
- a reservoir (F), which serves for taking up the gas exhaled by the patient (P),
- a pump (H), which serves for returning the exhaled gas that has been cleaned of carbon dioxide into the gas storage unit (B), and
- an analysis/control unit (J), which analyzes the gas returned to the gas storage unit (B) and, on the basis of its composition, triggers the metering device (A), are provided.

2. Device according to Claim 1, **characterized in that** a one-way valve (E), which prevents backflow of the gas exhaled by the patient (P), is arranged upstream of the reservoir (F).

3. Device according to Claim 2, **characterized in that** the carbon dioxide separating unit (G) is designed as a carbon dioxide separating unit that operates by absorption, adsorption and/or permeation.

4. Device according to one of the preceding Claims 1 to 3, **characterized in that** a flushing device (C), which serves for removing gas, is arranged upstream of the demand valve (D).

5. Device according to one of the preceding Claims 1 to 4, **characterized in that** the reservoir (F) is designed as a breathing pouch.

6. Device according to one of the preceding Claims 1 to 5, **characterized in that** the pump (H) has a safety circuit, which prevents the reservoir (F) from being emptied completely.

## Revendications

1. Dispositif pour délivrer un gaz à un patient, en particulier un patient respirant spontanément, le dispositif présentant :
un dispositif de dosage (A) qui sert à doser dans la réserve (B) à gaz le gaz que le patient (P) doit inhaler,
une réserve (B) à gaz qui sert à emmagasiner le gaz que le patient (P) doit inhaler,
une soupape de demande (D) qui régule le débit du gaz inhalé par le patient (P) et
une unité (G) de séparation de dioxyde de carbone qui sert à extraire le dioxyde de carbone du gaz expiré,
**caractérisé en ce que**
le dispositif présente un réservoir (F) qui sert à reprendre le gaz expiré par le patient (P),
une pompe (H) qui sert à renvoyer dans la réserve (B) à gaz le gaz expiré débarrassé du dioxyde de carbone et
une unité (J) d'analyse et/ou de commande qui analyse le gaz renvoyé dans la réserve (B) à gaz et qui commande le dispositif de dosage (A) en fonction de la composition de ce gaz.

2. Dispositif selon la revendication 1, **caractérisé en ce qu'**une soupape (E) à une voie qui empêche le retour du gaz expiré par le patient (P) est raccordée en amont du réservoir (F).

3. Dispositif selon la revendication 2, **caractérisé en ce que** l'unité (G) de séparation de dioxyde de carbone est configurée comme unité de séparation de dioxyde de carbone travaillant par absorption, par adsorption et/ou par perméation.

4. Dispositif selon l'une des revendications 1 à 3 qui précèdent, **caractérisé en ce qu'**un dispositif de balayage (C) qui sert à évacuer le gaz est raccordé en amont de la soupape de demande (D).

5. Dispositif selon l'une des revendications 1 à 4 qui précèdent, **caractérisé en ce que** le réservoir (F) est configuré comme sac respiratoire.

6. Dispositif selon l'une des revendications 1 à 5 qui précèdent, **caractérisé en ce que** la pompe (H) présente un circuit de protection qui empêche que le réservoir (F) se vide complètement.
